# EUROPEAN PATENT SPECIFICATION

(11) **EP 2 679 274 B1**
(45) Date of publication and mention of the grant of the patent: **23.01.2019**
(21) Application number: 13171465.1
(22) Date of filing: 11.06.2013
(51) Int. Cl.: A61M 39/26

(54) **Needle-free medicine injection connector without experiencing non-positive and non-negative pressure**
Nadelloser Medizininjektionssteckverbinder ohne Vorhandensein von nicht-positivem und nicht-negativem Druck
Connecteur d'injection d'un médicament sans aiguille sans subir de pression non positive et non négative

(30) Priority: 27.06.2012 BR 102012015897
(43) Date of publication of application: 01.01.2014
(73) Proprietor: Pan, Hsiu-Feng, New Taipei City (TW)
(72) Inventor: Tsai, Hsi-Chin, New Taipei City (TW); Chen, Wen-Chieh, New Taipei City (TW)
(74) Representative: Gee, Steven William

(56) References cited:
- US-A1- 2006 178 645
- US-A1- 2007 218 745
- US-A1- 2010 108 681
- US-A1- 2011 015 566
- US-B1- 6 189 859

## Description

### BACKGROUND OF THE INVENTION

### 1. Field of the Invention

The present invention relates to a connector, and more particularly to a needle-free syringe adapter that has superior hermetic characteristics to prevent backflow of liquid medicine and injection of air.

### 2. The Prior Arts

Needle syringes are common medical implements for injecting liquid medicine into patients. However, the needle syringes are dangerous and need recycling procedures that increase the cost of medical treatment.

Therefore, a needle-free syringe has been developed and may be connected to an adapter that is mounted on a cannula. The needle-free syringe has low hazard and may be reused to lower the cost of medical treatment.

A conventional connector for a needle syringe has a considerable vacant space and has an unsatisfied hermetic characteristic so that an appreciable amount of liquid medicine and air easily remain in the connector after an injection procedure is implemented. When the connector is connected to a needle-free syringe for another injection procedure, the aforementioned remaining liquid medicine and air are injected into the patient's blood vessel by a positive pressure difference between the adapter and the patient's inside. The patient's blood vessel is expanded inadvertently and causes pain. When the injection procedure is finished and the needle-free syringe is disconnected from the adapter, the blood and the liquid medicine in the blood vessel flow reversely backwards into the inner space of the adapter due to the negative pressure. Therefore, the inadvertent expansion during the aforementioned injection procedure makes patients feel pain. The reverse flow of the blood and the liquid medicine into the adapter repeatedly mixes with new liquid medicine in subsequent injection procedures to lower the curative effect of the liquid medicine and probably cause drug allergy.

US patent No. 8,277,424 discloses a needle-less syringe adapter. The piercing conduit has a planar top end and a planar bottom end. When the piercing conduit is inserted and assembled into the resilient valve, the resilient valve is easily scratched and even damaged by the planar top and bottom ends of the piercing conduit. Furthermore, the resilient valve is repeatedly extruded and contracted in the process of assembly and disassembly with the piercing conduit, so that the resilient valve is easily deformed even loosened from the piercing conduit.

US patent application 2011/0015566 discloses a needle-free medicine injection connector having the features of the preamble of claim 1. Other needle-free injection connectors are described in US 2010/0108681, US 6,189,859, US 2007/218745 and US 2006/178645.

To overcome the above shortcomings, the present invention provides a needle-free syringe connector to mitigate or obviate the aforementioned problems.

### SUMMARY OF THE INVENTION

A primary objective of the present invention is to provide a needle-free medicine injection connector that has superior hermetic characteristics to prevent backflow of the liquid medicine and injection of air which causes a patient to feel pain.

To achieve the above objectives, a needle-free medicine injection connector according to the present invention comprises: a covering part with an injection port, at least one air vent provided on the covering part, a positioning notch and a groove configured on an outer surface of the covering part; a connecting part with an inner cavity, the cavity having a bottom, a piercing passage defined through the connecting part, a hole defined at the bottom end, the connecting part having a positioning projection for engagement with the groove of the covering part, wherein the connecting part has a protruding part for engagement with the positioning notch of the covering part for preventing the connecting part from slippage or coming off when a syringe is attached with the injection port; and a valve unit installed in the cavity of the connecting part, the valve unit comprising a piercing conduit member and a resilient valve composed of a top half valve and a bottom half valve.

The top half valve has a first resilient part. One end of the first resilient part has a top surface, and a top slit is defined on the top surface of the top half valve. The top surface of the top half valve has a rim for sealing the injection port. The other end of the first resilient part has a first engaging portion having a first passage defined therethrough and communicated with the top slit. The first engaging portion of the top half valve has a first mounting part.

The bottom half valve has a second resilient part. One end of the second resilient part has a base, and a bottom slit is defined on the base of the bottom half valve. The other end of the second resilient part has a second engaging portion having a second passage defined therethrough and communicated with the bottom slit. The second engaging portion of the bottom half valve has a second mounting part.

The piercing conduit member is installed between the top half valve and the bottom half valve, the piercing conduit member has a main positioning part disposed between a first engaging portion of the top half valve and a second engaging portion of the bottom half valve, a flow passage is defined through the piercing conduit member, the flow passage has two openings at both ends thereof, a first opening at one end and a second opening at the other end thereof, the second opening is connected to the hole of the bottom of the connecting part.

When the needle-free syringe is not inserted into the injection port, the top half valve of the resilient valve seals the top injection opening. The top and bottom slits of the resilient valve are both closed with no air leakages. The closed state could prevent air or medicine backflow to patient's body. When the needle-free syringe is inserted into the top injection port of the covering part and compresses the top of the resilient valve, the top slit of the resilient valve is pierced and opened by the piercing conduit member and the flow passage of the piercing conduit member communicates with the needle-free syringe.

When the injection procedure is finished and the needle-free syringe is removed, the top and bottom slits recover to the closed state. The compressed top half valve and the bottom half valve recover to its original position along with the piercing conduit member.

Before the connection of the needle-free syringe, the resilient valve hermetically closes the top and bottom slits to prevent ambient air or liquid medicine and blood of the patient's blood vessel from flowing into the resilient valve. After the removal of the needle-free syringe, the compressed resilient valve recovers quickly along the slope of the piercing conduit member to immediately close the top and bottom slits to prevent any reverse flow of air, liquid medicine and blood. Therefore, the needle-free syringe adapter reduces the patient's pain which is caused by either negative or positive pressure. In addition, it prevents the possibility of patient's contamination.

When a user inserts a syringe into the injection port of the covering part and compresses the top surface, the top half valve of the resilient valve compresses the bottom half valve subsequently. In the meantime, the first resilient part and the second resilient part contract and make both of the top half valve and the bottom half valve shorter.

Under this situation, the openings of the piercing conduit member would come out of the top slit and the bottom slit through the first passage and the second passage. Therefore the second opening would connect the hole of the covering part. When this situation is present, an interflowing state is provided between the needle-free syringe, the piercing conduit member, and the covering part's passage.

Under this connective state, the user can inject medicine into catheter from the syringe and through the piercing conduit member, and make the dosed medicine effect better.

### BRIEF DESCRIPTION OF THE DRAWINGS

The present invention will be apparent to those skilled in the art by reading the following detailed description of a preferred embodiment thereof, with reference to the attached drawings, in which:
Fig. 1 is a perspective view of a needle-free medicine injection connector in accordance with the present invention;
Fig. 2 is an exploded perspective view of the needle-free medicine injection connector of the present invention ;
Fig. 3 is a cross-sectional view of the needle-free medicine injection connector of the present invention;
Fig. 4 is a cross-sectional view showing a syringe inserted into the needle-free medicine injection connector of the present invention; and
Fig. 5 is a perspective view of the piercing conduit member of the needle-free medicine injection connector in accordance with the present invention.

### DETAILED DESCRIPTION OF THE PREFERRED EMBODIMENT

The accompanying drawings are included to provide a further understanding of the invention, and are incorporated in and constitute a part of this specification. The drawings illustrate embodiments of the invention and, together with the description, serve to explain the principles of the invention.

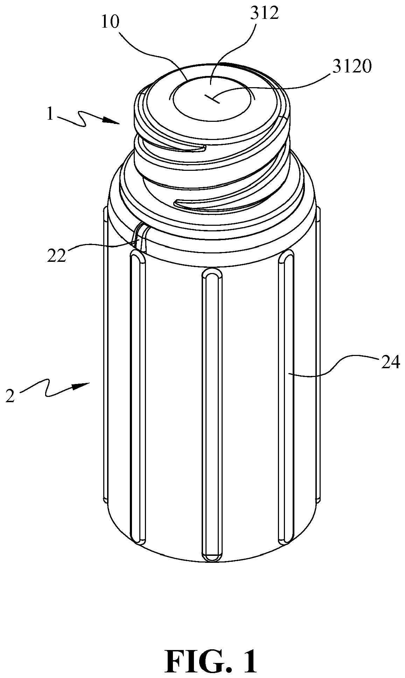

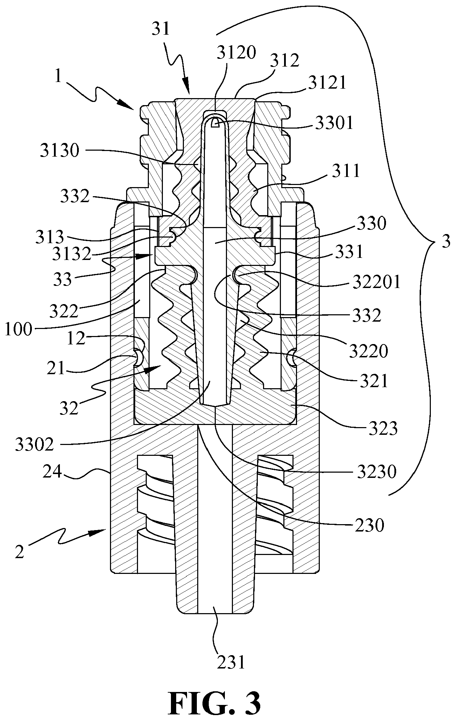

With reference to the attached Fig.1 to Fig.3, the needle-free medicine injection connector without experiencing non-positive and non-negative pressure includes: a covering part (1) with an injection port (10), and a connecting part (2) with an inner containable cavity .

The covering part (1) with the injection port (10) has a screw thread around (not identified) and has at least one air vent (100). The covering part has a positioning notch (11) which is a concave engaging structure and at least one groove (12). In addition, the covering part (1) has at least one concave track (13).

At least one end of the connecting part (2) has thread structure. On the outer surface of the connecting part (2) is the slip-proof bar (24) which are collateral protruding bars. The slip-proof bar (24) helps users not to slip when twisting the connecting part. The connecting part can be assembled with the covering part; containable cavity has a bottom part (23) which is pierced by a conduit (231); the conduit has a hole (230) at the bottom part. In the inner surface of the containable cavity, there is a protruding track (25) which engages with concave track (13). As a result, no slipping would happen when the covering part is assembled with the connecting part (2); the connecting part (2) has protruding circular lip (21) which connects with groove of the covering part. There is a protruding part (22) on connecting part which engages with the positioning notch (11) of the covering part (2) to prevent the connecting part (2) from slipping or coming off when a syringe is attached with the injection port (10). The connection of connecting part (2) can be a three type-Y type tube, a standalone type tube, or a thread structure type.

A valve unit (3) is installed in the cavity of the connecting part (2). It includes a piercing conduit member (33) and a resilient valve divided into a top half valve (31) and a bottom half valve (32).

The top half valve (31) has a first resilient part (311) which has crease structure and makes the top half valve (31) elastic. On the top surface (312) of the top half valve is a top slit (3120). The top surface has a rim (3121) which seals the injection port (10). On the other side it has the first engaging portion (313) which can communicate with the top slit.

When the rim (3121) rebounds back to its original location, it scrapes the remaining medicine liquid and prevents residue which might affect the following process. For the reason that the rim (3121) closely engages the inner wall, whereby perfect sealing effect can be performed. The top half valve has at least one first mounting part (3132). The first passage (3130) of the top half valve (31) has an exit at top slit (3120).

The bottom half valve (32) has a second resilient part (321) which has crease structure and makes the bottom half valve elastic constantly. Second resilient part (321) has a base (323) at one end and has an opening (3230) at its other end; the second engaging portion (322) has a second mounting part (32201). There is a flow passage (330) inside the piercing conduit member (33). The flow passage (330) is straight and has an opening at both ends, a first opening (3301) and a second opening (3302). The first opening (3301) has a dome end (33010) which has at least one side perforation (33011). The design of the dome end can prevent the first opening (3301) from causing damage when it shuttles through top slit (3120); the side opening (33011) can also draw liquid medicine when first opening (3301) sticks out of top slit (3120).

The piercing conduit member (33) is installed in between the first passage (3130) of the top half valve (31) and the second passage (3220) of the bottom half valve (32). The piercing conduit member has a main positioning part (331) set in-between the first engaging portion and the second engaging portion. The piercing conduit member also has at least one secondary positioning part (332) which can mount on the first mounting part (3132) of the top half valve (31) and the secondary mounting (32201) part of the bottom half valve (32). By this kind of mutual engaged structure, the piercing conduit member can be fixed with the top half valve (31) and the bottom half valve (32) tightly and prevent from coming loose.

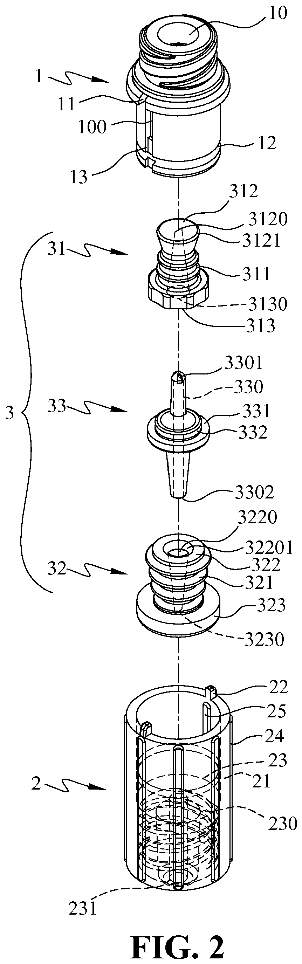

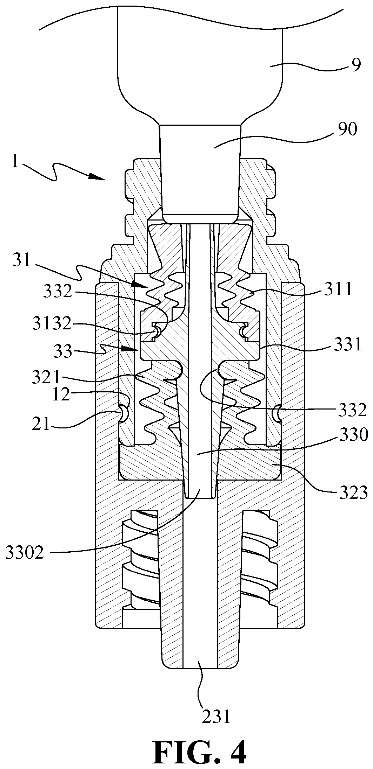

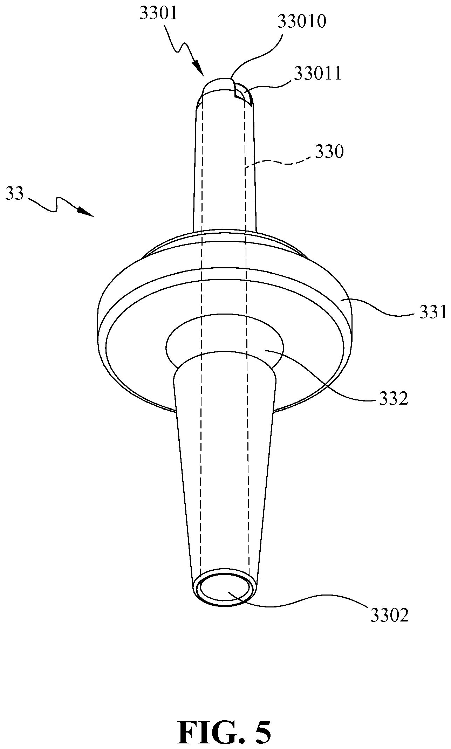

With reference to Figs. 2-5, the top half valve (31) and the bottom half valve (32) are made from resilient material. The resilient material could be silicon or other resilient objects. The piercing conduit member (33) mentioned above is made from relatively hard material.

When the present invention is set on the infusion set's catheter or the cap of an intravenous drip tube, the hospital personnel would be ready to inject medicine into patient with the present invention. Hospital personnel would connect the needle-free syringe (9) with the injection part (10) of the covering part (1), then the injector (90) presses the top surface (312) of the top half valve; the top half valve (31) would compress the bottom half valve (32) subsequently. As a result, first resilient part (311) of the top half valve (31) and the second resilient part (321) of the bottom half valve (32) contract and shorten under pressure and, in the meantime, the first opening (3301) of piercing conduit member (33) would drill through top slit (3120) along first passage (3130); the second opening (3302) of piercing conduit member (33) would drill through bottom slit (3230) along second passage (3220). At this point, the second opening (3302) connects with the hole (230) at the bottom part (23).

Therefore, a connective state is constructed by injector (90) of needle-free syringe (9), providing a flow passage (330) of piercing conduit member (33) and connecting part (2). At this moment, user could inject medicine into the injection port (10) from needle-free syringe (9), making liquid medicine flow to catheter through flow passage (330) of piercing conduit member (33) and conduit (231) of connecting part (2).

When the injector (90) screws onto the injection part (10) of the covering part (1), the air inside covering part (1) exhausts through air vent (100). On the contrary, when injector (90) is detached from injection part (10), the air outside flows in through air vent (100). Under this structure, no positive or negative air pressure would occur during attachment or detachment of injector (90) of syringe. In addition, there are indentations (not specified) along the first engaging portion (313). The indentation allows air to flow fluently inside covering part, preventing the first engaging portion (313) from rough motion caused by jammed air.

With reference to the structure, it is very easy to assemble and disassemble the present invention. Because the flow passage (330) of piercing conduit member (33) is straight, the injection is very smooth and hard to have jam, dysfunction or influent flow. Also it is easy to clean the present invention with little water, and it is suitable for repeated use which makes the medical cost decrease.

Before the needle-free syringe (9) is connected with present invention, the top half valve (31) and the bottom half valve (32) remain in the original form and are not deformed by outer force. Top slit (3120) and bottom slit (3230) remain in an airtight state which effectively prevents the backflow of medicine or blood inside patient's blood stream. After the needle-free syringe (9) is removed from the present invention, the vertically compressed top half valve (31) and bottom half valve (32) would come back to their original from along the piercing conduit member (33). Therefore the top slit (3120) and bottom slit (3230) get back to closure and an airtight state. As a result of the technology of no backflow, the present invention could significantly reduce pain feeling for patients.

In addition, the first passage's inner surface (3130) has contractive effect to prevent medicine from infiltrating into the first resilient part's inter space (311).

By means of the above mentioned special design of the valve unit (3), residual inner air, medicine or back flow could be reduced to the minimum degree. In addition, effective element of negative or positive pressure could be minimized to even zero to reduce the possibility of patient's infection.

In summary, the content of technology of the present invention conforms to the requirement of new patent, and it is practically used in medical industry now. Before the application, the present invention's technical feature is not published or used publicly. Further, the present invention effectively resolves the mid-term and long-term problem of previous medical technology, and it also meets needs of related users. Therefore it can be proven that this present invention's achievement is difficult to accomplish. It is apparent to those skilled in the art that the present invention is abundant in "industry usage", "innovation", and "advancement". We hereby respectfully ask for an allowance of this patent application, in order to protect the applicant's patent right.

Although the present invention has been described with reference to the preferred embodiments thereof, it is apparent to those skilled in the art that a variety of modifications and changes may be made without departing from the scope of the present invention which is intended to be defined by the appended claims.

## Claims

1. A needle-free medicine injection connector (4), comprising:
a covering part (1) with an injection port (10), a positioning notch (11) and a groove (12) configured on an out surface of the covering part;
a connecting part (2) with an inner cavity, the cavity having a bottom (23), a piercing passage (231) defined through the connecting part, a hole (230) defined at the bottom, the connecting part having a positioning projection (21) for engagement with the groove (12) of the covering part, wherein the connecting part has a protruding part (22) for engagement with the positioning notch (11) of the covering part for preventing the connecting part from slippage or coming off when a syringe (9) is attached with the injection port;
a valve unit (3) installed in the cavity of the connecting part, the valve unit comprising a piercing conduit member (33) and a resilient valve composed of a top half valve (31) and a bottom half valve (32), wherein
the top half valve has a first resilient part (311), one end of the first resilient part has a top surface (312), a top slit (320) is defined on the top surface of the top half valve, the top surface of the top half valve has a rim (321) for sealing the injection port (10), the other end of the first resilient part has a first engaging portion (313) having a first passage (3130) defined therethrough and communicating with the top slit, the first engaging portion (313) of the top half valve has a first mounting part (3132);
the bottom half valve has a second resilient part (321), one end of the second resilient part has a base (323), a bottom slit (3230) is defined on the base of the bottom half valve, the other end of the second resilient part has a second engaging portion (322) having a second passage (3220) defined therethrough and communicating with the bottom slit (3230), the second engaging portion of the bottom half valve has a second mounting part (32201);
the piercing conduit member (33) is installed between the top half valve and the bottom half valve, the piercing conduit member has a main positioning part (331) disposed between a first engaging portion (313) of the top half valve (31) and a second engaging portion (322) of the bottom half valve (32), a flow passage is defined through the piercing conduit member, the flow passage has an opening at both ends thereof, a first opening (3301) at one end and a second opening (3302) at the other end thereof, the second opening is connected to the hole (230) of the bottom of the connecting part
the injection connector having at least one air vent (100),
**characterised in that** the at least one air vent is an elongate slot through the wall of the covering part, the air vent extending between the groove (12) and the positioning notch (11) and being aligned with a concave track (13) of the covering part, **in that** the slot extends in the direction of movement of the piercing conduit member, and **in that** the air vent extends to both sides of the main positioning part (331).

2. The needle-free medicine injection connector as claimed in claim 1, wherein the protruding part (22) is engaged with the positioning notch (11); and the connecting part (2) has a protruding circle (21) engaged with the groove (12) of the covering part; and a protruding track (25) on an inner surface of the connecting part is engaged with the concave track (13) of the covering part.

3. The needle-free medicine injection connector as claimed in claim 1, wherein the first and second resilient parts (311, 321) each have a crease structure.

4. The needle-free medicine injection connector as claimed in claim 1, wherein a connection of the connecting part (2) is a three type-Y type, a standalone type, or a thread structure type.

5. The needle-free medicine injection connector as claimed in claim 1, wherein the top half valve (31) and the bottom half valve (32) are made from a resilient material, the resilient material is silicon, or other resilient materials; and the piercing conduit member (33) is made from a relatively hard material that is plastic or other hard materials.

6. The needle-free medicine injection connector as claimed in claim 1, wherein the piercing conduit member (33) has at least one secondary positioning part (332) engaged with the first mounting part (3132) of the top half valve (31) and the second mounting part (32201) of the bottom half valve (32).

7. The needle-free medicine injection connector as claimed in claim 1, wherein the flow passage (330) of the piercing conduit member (33) is straight.

8. The needle-free medicine injection connector as claimed in claim 1, wherein the piercing conduit member (33) is pillar shape or shuttle shape.

9. The needle-free medicine injection connector as claimed in claim 1, wherein the first opening (3301) has a dome end having at least one side perforation.

## Patentansprüche

1. Nadelloser Medizininjektionssteckverbinder (4), umfassend:
ein Abdeckteil (1) mit einem Injektionsanschluss (10), einer Positionierungskerbe (11) und einer Nut (12), die auf einer Außenfläche des Abdeckteils konfiguriert sind;
ein Verbindungsteil (2) mit einem inneren Hohlraum, der Hohlraum eine Unterseite (23) aufweisend, einer durch das Verbindungsteil definierten Durchsteckpassage (231), einem an der Unterseite definiertem Loch (230), das Verbindungsteil einen Positionierungsvorsprung (21) zum Eingreifen in die Nut (12) des Abdeckteils aufweisend, wobei das Verbindungsteil ein vorstehendes Teil (22) zum Eingreifen in die Positionierungskerbe (11) des Abdeckteils aufweist, um zu verhindern, dass das Verbindungsteil verrutscht oder sich löst, wenn eine Kanüle (9) mit dem Injektionsanschluss verbunden wird;
eine in dem Hohlraum des Verbindungsteils installierte Ventileinheit (3), die Ventileinheit ein Durchstichkanalelement (33) und ein elastisches Ventil umfassend, das aus einer oberen Ventilhälfte (31) und einer unteren Ventilhälfte (32) besteht, wobei
die obere Ventilhälfte ein erstes elastisches Teil (311) aufweist, ein Ende der ersten elastischen Teils eine Oberseite (312) aufweist, auf der Oberseite der oberen Ventilhälfte ein oberer Schlitz (320) definiert ist, die Oberseite der oberen Ventilhälfte einen Saum (321) zum Abdichten des Injektionsanschlusses (10) aufweist, das andere Ende des ersten elastischen Teils einen ersten eingreifenden Abschnitt (313) aufweist, der eine erste Passage (3130) aufweist, die durch diesen hindurch definiert ist und mit dem oberen Schlitz kommuniziert, der erste eingreifende Abschnitt (313) der oberen Ventilhälfte ein erstes Befestigungsteil (3132) aufweist;
die untere Ventilhälfte ein zweites elastisches Teil (321) aufweist, ein Ende des zweiten elastischen Teils eine Basis (323) aufweist, auf der Basis der unteren Ventilhälfte ein unterer Schlitz (3230) definiert ist, das andere Ende des zweiten elastischen Teils einen zweiten eingreifenden Abschnitt (322) aufweist, der eine zweite Passage (3220) aufweist, die durch diesen hindurch definiert ist und mit dem unteren Schlitz (3230) kommuniziert, der zweite eingreifende Abschnitt der unteren Ventilhälfte ein zweites Befestigungsteil (32201) aufweist;
das Durchstichkanalelement (33) zwischen der oberen Ventilhälfte und der unteren Ventilhälfte installiert ist, das Durchstichkanalelement ein Hauptpositionierungsteil (331) aufweist, das zwischen einem ersten eingreifenden Abschnitt (313) der oberen Ventilhälfte (31) und einen zweiten eingreifenden Abschnitt (322) der unteren Ventilhälfte (32) angeordnet ist, eine Durchflusspassage durch das Durchstichkanalelement hindurch definiert ist, die Durchflusspassage eine Öffnung an ihren beiden Enden aufweist, eine erste Öffnung (3301) an ihrem einen Ende und eine zweite Öffnung (3302) an ihrem anderen Ende, wobei die zweite Öffnung mit dem Loch (230) der Unterseite des Verbindungsteils verbunden ist,
der Injektionssteckverbinder mindestens eine Entlüftungsöffnung (100) aufweisend,
**dadurch gekennzeichnet, dass** die mindestens eine Entlüftungsöffnung ein länglicher Schlitz durch die Wand des Abdeckteils hindurch ist, die Entlüftungsöffnung zwischen der Nut (12) und der Positionierungskerbe (11) verläuft und an einer konkaven Bahn (13) des Abdeckteils ausgerichtet ist, dass der Schlitz in der Bewegungsrichtung des Durchstechkanalelements verläuft und dass die Entlüftungsöffnung zu beiden Seiten des Hauptpositionierungsteils (331) verläuft.

2. Nadelloser Medizininjektionssteckverbinder nach Anspruch 1, wobei das vorstehende Teil (22) in die Positionierungskerbe (11) eingreift; und das Verbindungsteil (2) einen vorstehenden Kreis (21) aufweist, der in die Nut (12) des Abdeckteils eingreift; und eine vorstehende Bahn (25) auf einer Innenfläche des Verbindungsteils in die konkave Bahn (13) des Abdeckteils eingreift.

3. Nadelloser Medizininjektionssteckverbinder nach Anspruch 1, wobei die ersten und zweiten elastischen Teile (311, 321) jeweils eine Faltenstruktur aufweisen.

4. Nadelloser Medizininjektionssteckverbinder nach Anspruch 1, wobei eine Verbindung des Verbindungsteils (2) ein Dreityp-Y-Typ, ein eigenständiger Typ oder ein Gewindestruktur-Typ ist.

5. Nadelloser Medizininjektionssteckverbinder nach Anspruch 1, wobei die obere Ventilhälfte (31) und die untere Ventilhälfte (32) aus einem elastischen Material bestehen, wobei das elastische Material Silikon ist, oder aus anderen elastischen Materialien; und das Durchstichkanalelement (33) aus einem relativ harten Material besteht, das Kunststoff ist, oder aus anderen harten Materialien.

6. Nadelloser Medizininjektionssteckverbinder nach Anspruch 1, wobei das Durchstichkanalelement (33) mindestens ein sekundäres Positionierungsteil (332) aufweist, das in das erste Befestigungsteil (3132) der oberen Ventilhälfte (31) und das zweite Befestigungsteil (32201) der unteren Ventilhälfte (32) eingreift.

7. Nadelloser Medizininjektionssteckverbinder nach Anspruch 1, wobei die Durchflusspassage (330) des Durchstichkanalelements (33) gerade ist.

8. Nadelloser Medizininjektionssteckverbinder nach Anspruch 1, wobei das Durchstichkanalelement (33) eine Säulenform oder Schiffchenform aufweist.

9. Nadelloser Medizininjektionssteckverbinder nach Anspruch 1, wobei die erste Öffnung (3301) ein Kuppelende aufweist, das mindestens eine Seitenperforation aufweist.

## Revendications

1. Un connecteur d'injection de médicament sans aiguille (4), comprenant :
une partie de couverture (1) avec un orifice d'injection (10), une encoche de positionnement (11) et une rainure (12) configurés sur une surface extérieure de la partie de couverture ;
une partie de connexion (2) avec une cavité interne, la cavité ayant un fond (23), un passage de perçage (231) défini à travers la partie de connexion, un trou (230) défini au fond, la partie de connexion ayant une projection de positionnement (21) pour une mise en prise avec la rainure (12) de la partie de couverture, la partie de connexion ayant une partie en saillie (22) pour une mise en prise avec l'encoche de positionnement (11) de la partie de couverture pour empêcher la partie de connexion de glisser ou de se détacher lorsqu'une seringue (9) est attachée à l'orifice d'injection ;
une unité de valve (3) installée dans la cavité de la partie de connexion, l'unité
de valve comprenant un élément formant conduit de perçage (33) et une valve élastique composée d'une demi-valve supérieure (31) et d'une demi-valve inférieure (32), dans lequel
la demi-valve supérieure a une première partie élastique (311), une extrémité de la première partie élastique a une surface supérieure (312), une fente supérieure (320) est définie sur la surface supérieure de la demi-valve supérieure, la surface supérieure de la demi-valve supérieure a un rebord (321) pour fermer hermétiquement l'orifice d'injection (10), l'autre extrémité de la première partie élastique a une première portion de mise en prise (313) ayant un premier passage (3130) défini à travers celle-ci et communiquant avec la fente supérieure, la première portion de mise en prise (313) de la demi-valve supérieure a une première partie de montage (3132) ;
la demi-valve inférieure a une deuxième partie élastique (321), une extrémité de la deuxième partie élastique a une base (323), une fente inférieure (3230) est définie sur la base de la demi-valve inférieure, l'autre extrémité de la deuxième partie élastique a une deuxième portion de mise en prise (322) ayant un deuxième passage (3220) défini à travers celle-ci et communiquant avec la fente inférieure (3230), la deuxième portion de mise en prise de la demi-valve inférieure a une deuxième partie de montage (32201) ;
l'élément formant conduit de perçage (33) est installé entre la demi-valve supérieure et la demi-valve inférieure, l'élément formant conduit de perçage a une partie de positionnement principale (331) disposée entre une première portion de mise en prise (313) de la demi-valve supérieure (31) et une deuxième portion de mise en prise (322) de la demi-valve inférieure (32), un passage d'écoulement est défini à travers l'élément formant conduit de perçage, le passage d'écoulement a une ouverture aux deux extrémités de celui-ci, une première ouverture (3301) à une extrémité et une deuxième ouverture (3302) à l'autre extrémité de celui-ci, la deuxième ouverture est connectée au trou (230) du fond de la partie de connexion
le connecteur d'injection ayant au moins un aérateur (100),
**caractérisé en ce que** l'au moins un aérateur est une fente allongée à travers la paroi de la partie de couverture, l'aérateur s'étendant entre la rainure (12) et l'encoche de positionnement (11) et étant aligné avec une glissière concave (13) de la partie de couverture, **en ce que** la fente s'étend dans la direction de déplacement de l'élément formant conduit de perçage, et **en ce que** l'aérateur s'étend des deux côtés de la partie de positionnement principale (331).

2. Le connecteur d'injection de médicament sans aiguille tel que revendiqué dans la revendication 1, dans lequel la partie en saillie (22) est en prise avec l'encoche de positionnement (11) ; et la partie de connexion (2) a un cercle en saillie (21) en prise avec la rainure (12) de la partie de couverture ; et une glissière en saillie (25) sur une surface interne de la partie de connexion est en prise avec la glissière concave (13) de la partie de couverture.

3. Le connecteur d'injection de médicament sans aiguille tel que revendiqué dans la revendication 1, dans lequel les première et deuxième parties élastiques (311, 321) ont chacune une structure en pli.

4. Le connecteur d'injection de médicament sans aiguille tel que revendiqué dans la revendication 1, dans lequel une connexion de la partie de connexion (2) est un type de type Y trois types, un type autonome, ou un type de structure filetée.

5. Le connecteur d'injection de médicament sans aiguille tel que revendiqué dans la revendication 1, dans lequel la demi-valve supérieure (31) et la demi-valve inférieure (32) sont fabriqués à partir d'un matériau élastique, le matériau élastique étant du silicium, ou d'autres matériaux élastiques ; et l'élément formant conduit de perçage (33) est fabriqué à partir d'un matériau relativement dur qui est du plastique ou autres matériaux durs.

6. Le connecteur d'injection de médicament sans aiguille tel que revendiqué dans la revendication 1, dans lequel l'élément formant conduit de perçage (33) a au moins une partie de positionnement secondaire (332) en prise avec la première partie de montage (3132) de la demi-valve supérieure (31) et la deuxième partie de montage (32201) de la demi-valve inférieure (32).

7. Le connecteur d'injection de médicament sans aiguille tel que revendiqué dans la revendication 1, dans lequel le passage d'écoulement (330) de l'élément formant conduit de perçage (33) est droit.

8. Le connecteur d'injection de médicament sans aiguille tel que revendiqué dans la revendication 1, dans lequel l'élément formant conduit de perçage (33) est en forme de pilier ou en forme de furet.

9. Le connecteur d'injection de médicament sans aiguille tel que revendiqué dans la revendication 1, dans lequel la première ouverture (3301) a une extrémité en dôme ayant au moins une perforation latérale.
